# EUROPEAN PATENT SPECIFICATION

(11) **EP 2 353 557 B1**
(45) Date of publication and mention of the grant of the patent: **27.05.2020**
(21) Application number: 10196887.3
(22) Date of filing: 26.02.2003
(51) Int. Cl.: A61B 17/12, A61F 11/00, A61B 17/22

(54) **Removable anchored lung volume reduction devices**
Entfernbare verankerte Lungenvolumen- Reduktionsvorrichtungen
Dispositifs de réduction du volume pulmonaire ancres et détachables

(30) Priority: 20.03.2002 US 103487; 17.05.2002 US 150547
(43) Date of publication of application: 10.08.2011
(62) Divisional of application: 03716212.0
(73) Proprietor: Spiration, Inc., Redmond, WA 98052 (US)
(72) Inventor: Alferness, Clifton, A., Redmond, WA 98053 (US); Dillard, David, H., Grapeview, WA 98546 (US); Gonzalez, Hugo, X., Woodinville, WA 98072 (US); Yi, Seung, Mission Viejo, CA 92691 (US); Devore, Lauri, J., Seattle, WA 98155 (US); Park, Mia, Redmond, WA 98052 (US); Corcoran, Dean, T., Bothell, WA 98012 (US); Rimbaugh, Jenni, Redmond, WA 98052 (US)
(74) Representative: Noack, Andreas

(56) References cited:
- WO-A-01/66190
- WO-A-01/87170
- GB-A- 2 324 729
- US-B1- 6 258 100

## Description

### Background

The present invention is generally directed to a removable anchored device for treating Chronic Obstructive Pulmonary Disease (COPD). The present invention is more particularly directed to providing an anchored intra-bronchial obstruction that may be removable.

COPD has become a major cause of morbidity and mortality in the United States over the last three decades. COPD is characterized by the presence of airflow obstruction due to chronic bronchitis or emphysema. The airflow obstruction in COPD is due largely to structural abnormalities in the smaller airways. Important causes are inflammation, fibrosis, goblet cell metaplasia, and smooth muscle hypertrophy in terminal bronchioles.

The incidence, prevalence, and health-related costs of COPD are on the rise. Mortality due to COPD is also on the rise. In 1991, COPD was the fourth leading cause of death in the United States and had increased 33% since 1979. COPD affects the patient's whole life. It has three main symptoms: cough; breathlessness; and wheeze. At first, breathlessness may be noticed when running for a bus, digging in the garden, or walking uphill. Later, it may be noticed when simply walking in the kitchen. Over time, it may occur with less and less effort until it is present all of the time. COPD is a progressive disease and currently has no cure. Current treatments for COPD include the prevention of further respiratory damage, pharmacotherapy, and surgery. Each is discussed below.

The prevention of further respiratory damage entails the adoption of a healthy lifestyle. Smoking cessation is believed to be the single most important therapeutic intervention. However, regular exercise and weight control are also important. Patients whose symptoms restrict their daily activities or who otherwise have an impaired quality of life may require a pulmonary rehabilitation program including ventilatory muscle training and breathing retraining. Long-term oxygen therapy may also become necessary.

Pharmacotherapy may include bronchodilator therapy to open up the airways as much as possible or inhaled beta-agonists. For those patients who respond poorly to the foregoing or who have persistent symptoms, ipratropium bromide may be indicated. Further, courses of steroids, such as corticosteroids, may be required. Lastly, antibiotics may be required to prevent infections and influenza and pneumococcal vaccines may be routinely administered. Unfortunately, there is no evidence that early, regular use of pharmacotherapy will alter the progression of COPD.

About 40 years ago, it was first postulated that the tethering force that tends to keep the intrathoracic airways open was lost in emphysema and that by surgically removing the most affected parts of the lungs, the force could be partially restored. Although the surgery was deemed promising, the lung volume reduction surgery (LVRS) procedure was abandoned. LVRS was later revived. In the early 1990's, hundreds of patients underwent the procedure. However, the procedure has fallen out of favor when Medicare stopping reimbursing for LVRS. Unfortunately, data is relatively scarce and many factors conspire to make what data exists difficult to interpret. The procedure is currently under review in a controlled clinical trial. However, what data does exist tends to indicate that patients benefited from the procedure in terms of an increase in forced expiratory volume, a decrease in total lung capacity, and a significant improvement in lung function, dyspnea, and quality of life. Improvements in pulmonary function after LVRS have been attributed to at least four possible mechanisms. These include enhanced elastic recoil, correction of ventilation/perfusion mismatch, improved efficiency of respiratory musculature, and improved right ventricular filling.

Lastly, lung transplantation is also an option. Today, COPD is the most common diagnosis for which lung transplantation is considered. Unfortunately, this consideration is given for only those with advanced COPD. Given the limited availability of donor organs, lung transplant is far from being available to all patients.

There is a need for additional non-surgical options for permanently treating COPD without surgery. A promising new therapy includes non-surgical apparatus and procedures for lung volume reduction by permanently obstructing the air passageway that communicates with the portion of the lung to be collapsed. The therapy includes placing an obstruction in the air passageway that prevents inhaled air from flowing into the portion of the lung to be collapsed. Lung volume reduction with concomitant improved pulmonary function may be obtained without the need for surgery. The effectiveness of obstructions may be enhanced if it is anchored in place. The effectiveness may also be enhanced if the obstruction is removable. However, no readily available apparatus and method exists for anchoring the obstruction, and for removal if required.

WO 01/66190 A2 discloses an implantable flow control device for insertion into an air passageway of a patient , the device comprises a one-way valve as flow control member and anchors for piercing a wall of the air passageway. WO 01/87170 A1 relates to a bronchio-pulmonary obstruction device with a tapered tubular sleeve, a flutter valve and a frame, barbs anchor the device in position. US 6,258,100 B1 pertains to an obstruction device for lung reduction having a hollow cylindrical configuration exhibiting an inner resilient reinforcement rib an having a one-way valve. In view of the foregoing, there is a need in the art for a new and improved apparatus and method for permanently obstructing an air passageway that is anchored in place, and that may be removed if required. The present invention is directed to a device that provides such an improved apparatus and method for treating COPD.

### Summary

The invention is disclosed by independent claim 1 and preferred embodiments are disclosed in the dependent claims. The present invention provides an anchored intra-bronchial device for placement in an air passageway of a patient to collapse a lung portion associated with the air passageway. The device includes an obstructing member that prevents air from being inhaled into the lung portion to collapse the lung portion, and an anchor that anchors the obstruction device within the air passageway when the anchor is deployed. The anchor engages the air passageway. The obstructing member and the anchor may be simultaneously deployable. The anchor may be releasable from the air passageway for removal of the obstructing member. A portion of the intra-bronchial device may be collapsible. The anchor may be releasable from the air passageway for removal of the obstructing member by collapsing a portion of the obstructing member, or by drawing the obstructing member proximally. The anchor may include a resilient material for imparting a force against the air passageway to deform the air passageway to more positively anchor the obstructing member. The anchor may comprise material having memory of an original shape, and resiliency to return the material to that shape. The obstructing member may comprise material having memory of an original shape, and resiliency to return the material to that shape. The obstructing member may be a one-way valve.

In another exemplary embodiment of the present disclosure, an exemplary method of reducing the size of a lung by collapsing a portion of the lung is provided. The exemplary method includes the step of providing an intra-bronchial device comprising an obstructing member which is so dimensioned when deployed in an air passageway communicating with the portion of the lung to be collapsed to preclude air from being inhaled, and an anchor that anchors the obstructing member when the anchor is deployed. The method further includes the steps of placing the obstructing member in the air passageway, and deploying the anchor. The anchor may be releasable for removal of the obstructing member. The obstructing member may form a one-way valve. A portion of the obstructing member may be collapsible.

In a further exemplary embodiment of the present disclosure an exemplary method of reducing the size of a lung by collapsing a portion of the lung with a removable device is provided. The method includes the step of providing an intra-bronchial device and an obstructing member that is so dimensioned when deployed in an air passageway communicating with the portion of the lung to be collapsed to preclude air from being inhaled, and an anchor that anchors the obstructing member when the anchor is deployed. The method includes the additional steps of placing the obstructing member in the air passageway, deploying an anchor, and removing the obstructing member. The anchor is releasable from the air passageway for removal of the intra-bronchial device, and the step of removing the obstructing member includes the further step of releasing the anchor. The obstructing member may form a one-way valve. At least a portion of the obstructing member may be collapsible, and the step of removing the obstructing member includes the further step of collapsing a portion of the obstructing member.

These and various other features as well as advantages which characterize the present invention will be apparent from a reading of the following detailed description and a review of the associated drawings.

### Brief Description of the Drawings

The features of the present invention which are believed to be novel are set forth with particularity in the appended claims. The invention, together with further objects and advantages thereof, may best be understood by making reference to the following description taken in conjunction with the accompanying drawings, in the several figures of which like referenced numerals identify like elements, and wherein:
Figure 1 is a simplified sectional view of a thorax illustrating a healthy respiratory system;
Figure 2 is a sectional view similar to FIG. 1, but illustrating a respiratory system suffering from COPD and the execution of a first step in treating the COPD condition by reducing the size of a lung;
Figure 3 is perspective view, partially in section, and to an enlarged scale, illustrating an intermediate step in the treatment;
Figure 4 is a perspective view of a conduit that may be utilized during the treatment;
Figure 5 is a perspective view of an exemplary intra-bronchial device, with anchors located proximally on peripheral portions of the support members, as the device would appear when fully deployed in an air passageway in accordance with the present disclosure;
Figure 6 is a partial section view of the device of FIG. 5 showing additional details of the support structure;
Figure 7 is a perspective view of the intra-bronchial device of FIG. 5 anchored in an air passageway;
Figure 8 illustrates another exemplary intra-bronchial device, with anchors carried distally on the central support structure, fully deployed in an air passageway in accordance with an alternative embodiment of the disclosure;
Figure 9 is a perspective view of another exemplary intra-bronchial device, with proximal anchors carried on the central support structure, in accordance with an alternative exemplary embodiment of the disclosure;
Figure 10 is of an exemplary intra-bronchial device, with proximal anchors carried on the central support structure, in accordance with an alternative exemplary embodiment of the disclosure;
Figure 11 is and view of an exemplary intra-bronchial device, with proximal anchors carried on the central support structure, in accordance with an alternative exemplary embodiment of the disclosure;
Figure 12 is a perspective view of an intra-bronchial device, with distal friction anchors carried on the central support structure, in accordance with an embodiment of the invention;
Figure 13 is a side view of the intra-bronchial device, of figure 12, with distal friction anchors carried on the central support structure, in accordance with an embodiment of the invention;
Figure 14 is an end view of the intra-bronchial device of figure 12, with distal friction anchors carried on the central support structure, in accordance with an embodiment of the invention;
Figure 15 is a perspective view an exemplary intra-bronchial device similar to that of FIGS. 12-14 anchored in an air passageway;
Figure 16 is a perspective view illustrating an alternative exemplary embodiment of a removable intra-bronchial device with proximal anchors carried on a peripheral portion of a plurality of support structure members in accord with the present disclosure;
Figure 17 is a side view of the device of Figure 16;
Figure 18 is a perspective view of an exemplary device in its deployed state with anchors carried on an obstructing member, in accordance with an alternative exemplary embodiment of the disclosure;
Figure 19 is a partial longitudinal sectional view of the device of FIG. 18 in a collapsed state and located into a lumen for placement in an air passageway;
Figure 20 is a perspective view of the device of FIG. 18 in its deployed and anchored state in an air passageway;
Figure 21 is a side view of an initial step in removing the device of FIG. 18 from an air passageway;
Figure 22 is a side view of an intermediate step in removing the device of FIG. 18 from an air passageway;
Figure 23 is a side view of another intermediate step in removing the device of FIG. 18 from an air passageway;
Figure 24 is a side view illustrating the collapse of the device of FIG. 18 during its removal from an air passageway;
Figure 25 is a perspective view of an exemplary device in its deployed state with anchors carried on the obstructing member, in accordance with an alternative embodiment of the present disclosure;
Figure 26 illustrates the placement and securing of the obstructing member of the device of FIG. 25 to a support member; and
Figure 27 is a perspective view of the exemplary intra-bronchial device of FIG. 25 fully deployed and anchored in an air passageway, in accordance with the present disclosure.

### Detailed Description

In the following detailed description of exemplary embodiments of the disclosure and embodiments according to the invention, reference is made to the accompanying drawings that form a part hereof. The detailed description and the drawings illustrate how specific exemplary embodiments and embodiments of the invention may be practiced. These embodiments are described in sufficient detail to enable those skilled in the art to practice the invention. It is understood that other embodiments may be utilized, and other changes may be made, without departing from the scope of the present invention. The following detailed description is therefore not to be taken in a limiting-sense, and the scope of the present invention is defined by the appended claims.

Throughout the specification and claims, the following terms take the meanings explicitly associated herein unless the context clearly dictates otherwise. The meaning of "a", "an", and "the" include plural references. The meaning of "in" includes "in" and "on." Referring to the drawings, like numbers indicate like parts throughout the views. Additionally, a reference to the singular includes a reference to the plural unless otherwise stated or inconsistent with the disclosure herein.

Additionally, throughout the specification, claims, and drawings, the term "proximal" means nearest the trachea, and "distal" means nearest the bronchioles.

Briefly stated, an aspect of the invention provides an anchored intra-bronchial device for placement in an air passageway of a patient to collapse a lung portion associated with the air passageway. A further aspect of the invention provides removability of the intra-bronchial device, either by releasing the anchors for removal of the entire device or by separating the obstructing member and removing it.

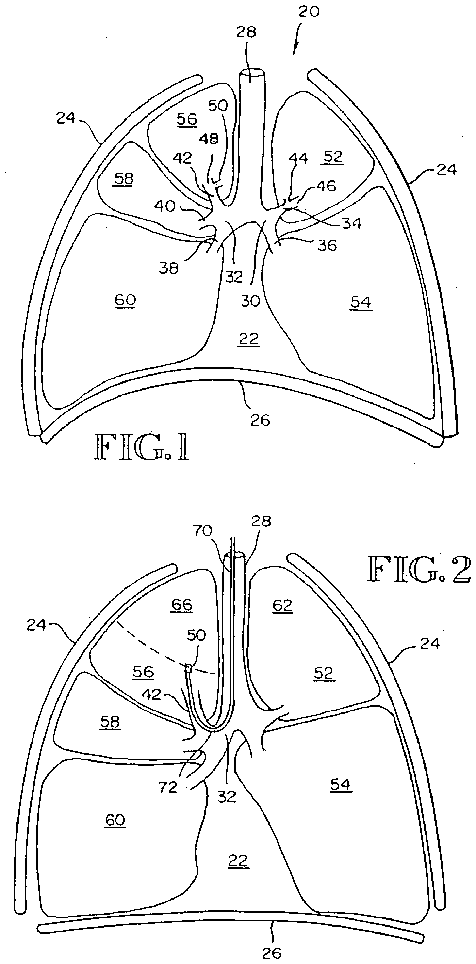

FIG. 1 is a sectional view of a healthy respiratory system. The respiratory system 20 resides within the thorax 22 that occupies a space defined by the chest wall 24 and the diaphragm 26.

The respiratory system 20 includes the trachea 28, the left mainstem bronchus 30, the right mainstem bronchus 32, the bronchial branches 34, 36, 38, 40, and 42 and sub-branches 44, 46, 48, and 50. The respiratory system 20 further includes left lung lobes 52 and 54 and right lung lobes 56, 58, and 60. Each bronchial branch and sub-branch communicates with a respective different portion of a lung lobe, either the entire lung lobe or a portion thereof. As used herein, the term "air passageway" is meant to denote either a bronchi or bronchiole, and typically means a bronchial branch or sub-branch which communicates with a corresponding individual lung lobe or lung lobe portion to provide inhaled air thereto or conduct exhaled air therefrom.

Characteristic of a healthy respiratory system is the arched or inwardly arcuate diaphragm 26. As the individual inhales, the diaphragm 26 straightens to increase the volume of the thorax 22. This causes a negative pressure within the thorax. The negative pressure within the thorax in turn causes the lung lobes to fill with air. When the individual exhales, the diaphragm returns to its original arched condition to decrease the volume of the thorax. The decreased volume of the thorax causes a positive pressure within the thorax which in turn causes exhalation of the lung lobes.

In contrast to the healthy respiratory system of FIG. 1, FIG. 2 illustrates a respiratory system suffering from COPD. Here it may be seen that the lung lobes 52, 54, 56, 58, and 60 are enlarged and that the diaphragm 26 is not arched but substantially straight. Hence, this individual is incapable of breathing normally by moving diaphragm 28. Instead, in order to create the negative pressure in thorax 22 required for breathing, this individual must move the chest wall outwardly to increase the volume of the thorax. This results in inefficient breathing causing these individuals to breathe rapidly with shallow breaths.

It has been found that the apex portions 62 and 66 of the upper lung lobes 52 and 56, respectively, are most affected by COPD. Hence, bronchial sub-branch obstructing devices are generally employed for treating the apex 66 of the right, upper lung lobe 56. However, as will be appreciated by those skilled in the art, the present invention may be applied to any lung portion without departing from the present invention. As will be further appreciates by those skilled the in art, the present invention may be used with any type of obstructing member to provide an anchored obstructing device, which may be removed. The inventions disclosed and claimed in United States Patent Numbers US 6,258,100 and US6,293,951, provide an improved therapy for treating COPD by obstructing an air passageway using an intra-bronchial valve or plug. The present invention may be used with the apparatus, system, and methods of these patents as will be briefly described in conjunction with the disclosure of the preferred embodiments.

The insertion of an obstructing member treats COPD by deriving the benefits of lung volume reduction surgery without the need of performing the surgery. The treatment contemplates permanent collapse of a lung portion. This leaves extra volume within the thorax for the diaphragm to assume its arched state for acting upon the remaining healthier lung tissue. As previously mentioned, this should result in improved pulmonary function due to enhanced elastic recoil, correction of ventilation/perfusion mismatch, improved efficiency of respiratory musculature, and improved right ventricle filling. The present invention supports the use of intra-bronchial plugs to treat COPD by anchoring the obstruction device in the air passageway. The present invention further supports the use of intra-bronchial plugs by providing for their removal if necessary. Use of anchors can allow the obstructing member to be relatively loosely fitted against the air passageway wall, which may provide increased mucociliary transport of mucus and debris out of the collapsed lung portion.

FIG. 2 also illustrates a step in an exemplary COPD treatment using an obstructing member. Treatment is initiated by feeding a conduit or catheter 70 down the trachea 28, into the right mainstem bronchus 32, into the bronchial branch 42 and into and terminating within the sub-branch 50. The sub-branch 50 is the air passageway that communicates with the lung portion 66 to be treated, and is also referred to herein as air passageway 50. The catheter 70 is preferably formed of flexible material such as polyethylene. Also, the catheter 70 is preferably preformed with a bend 72 to assist the feeding of the catheter from the right mainstem bronchus 32 into the bronchial branch 42.

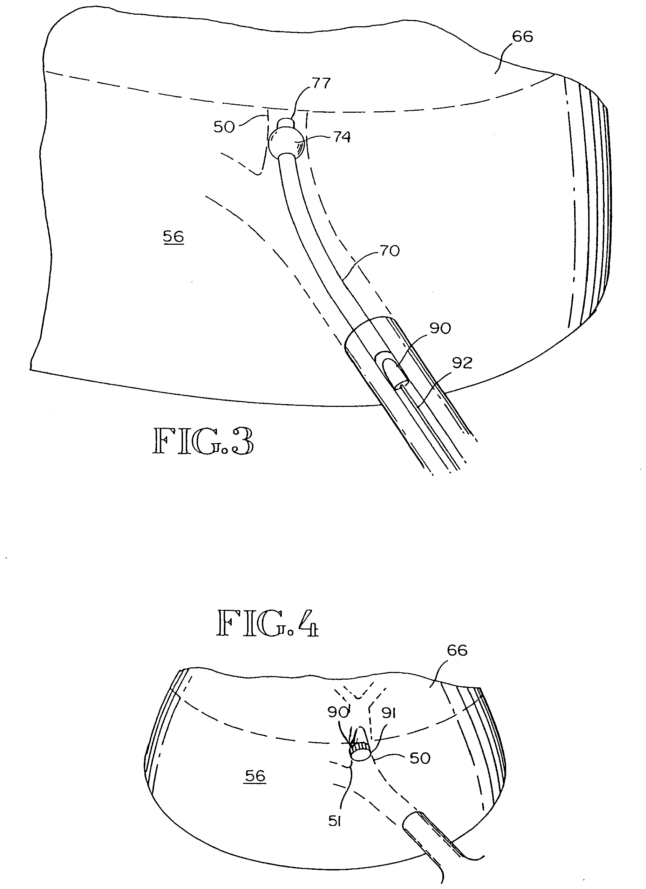

FIG. 3 illustrates a further step in an exemplary method for placing an obstructing member 90 in a bronchial sub-branch using a catheter. The invention disclosed herein is not limited to use with the particular exemplary method illustrated herein. Catheter 70 includes an optional inflatable sealing member 74 for use with a vacuum to collapse lung portion 66 prior to insertion of obstructing member 90. The obstructing member 90 may be formed of resilient or collapsible material to enable the obstructing member 90 to be fed through the catheter 70 in a collapsed state. The obstructing member 90 and its anchors (not shown) are collapsed and fed into the catheter 70. The stylet 92 is used to push the obstructing member 90 to the end 77 of the catheter 70 for placing the obstructing member 90 within the air passageway 50 adjacent to the lung portion 66 to be permanently collapsed. Optional sealing member 74 is withdrawn after obstructing member 90 is inserted.

A function of the intra-bronchial device disclosed and claimed in this specification, including the detailed description and the claims, is described in terms of collapsing a lung portion associated with an air passageway. In some lungs, a portion of a lung may receive air from collateral air passageways. Obstructing one of the collateral air passageways may reduce the volume of the lung portion associated with the air passageway, but not completely collapse the lung portion as that term may be generally understood. As used herein, the meaning of "collapse" includes both a complete collapse of a lung portion and a partial collapse of a lung portion.

Once deployed, the obstructing member precludes inhaled air from entering the lung portion to be collapsed. In accordance with the present invention, it is preferable that the obstructing member takes the form of a one-way valve. In addition to precluding inhaled air from entering the lung portion, the member further allows air within the lung portion to be exhaled. This results in more rapid collapse of the lung portion. In addition, anchoring obstructing members that preclude both inhaled and exhaled airflow are contemplated as within the scope of the disclosure.

FIG. 4 illustrates the obstructing device in place within air passageway 50. Obstructing member 90 has expanded upon placement in the air passageway 50 to seal the air passageway 50. This causes the lung portion 66 to be maintained in a permanently collapsed state. The obstructing member 90 may be any shape suitable for accomplishing its purpose, and may be a solid material or a membrane.

More specifically, the exemplary obstructing member 90 has an outer dimension 91, and when expanded, enables a contact zone with the air passageway inner dimension 51. This seals the air passageway upon placement of the obstructing member 90 in the air passageway 50 for maintaining the lung portion 66 in the collapsed state.

Alternatively, the lung portion 66 may be collapsed using vacuum prior to placement of obstructing member 90, or sealing the air passageway 50 with obstructing member 90 may collapse it. Over time, the air within the lung portion 66 will be absorbed by the body and result in the collapse of lung portion 66. Alternatively, obstructing member 90 may include the function of a one-way valve that allows air to escape from lung portion 66. Lung portion 66 will then collapse, and the valve will prevent air from being inhaled.

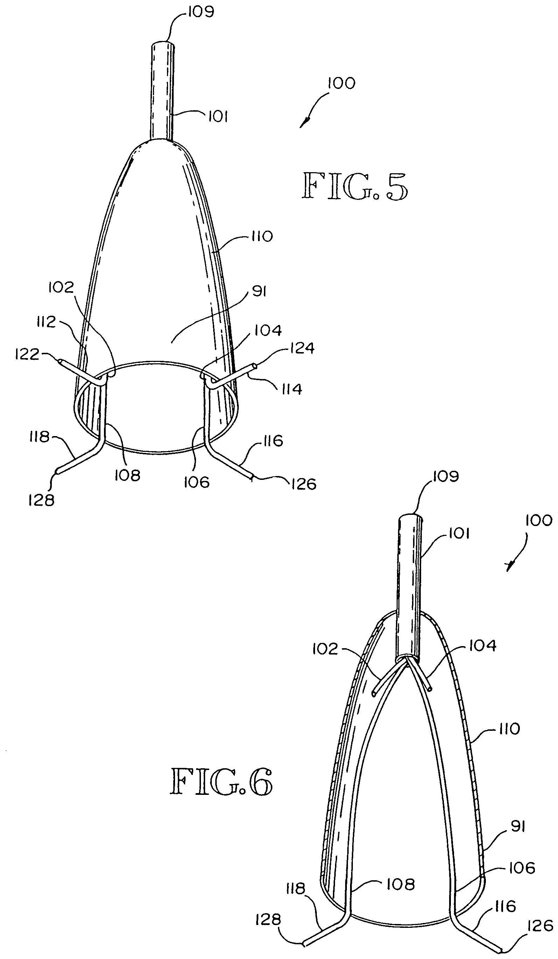

FIG. 5 is a perspective view of an exemplary intra-bronchial device, with anchors located proximally on peripheral portions of the support members, as the device would appear when fully deployed in an air passageway. Intra-bronchial device 100 includes a support structure 101, a central support structure 109; support members 102, 104, 106, and 108; anchors 112, 114, 116, and 118; anchor ends 122, 124, 126, and 128; and an obstructing member 110.

The support structure 101 of intra-bronchial device includes central support structure 109, and support members 102, 104, 106, and 108. The support members 102, 104, 106, and 108, carry anchors 112, 114, 116, and 118; and anchor ends 122, 124, 126, and 128, respectively. Central support structure 109 is a tubular member, preferably hypodermic needle tubing. Support members 102, 104, 106, and 108, are coupled mechanically to central support structure 109, such as by crimping, or by other methods such as adhesive or welding. Support members 102, 104, 106, and 108 are generally similar to each other. The support members are preferably formed of stainless steel, Nitinol, or other suitable material having a memory of its original shape, and resiliency to return the material to that shape.

Anchors 112, 114, 116, and 118 are extensions of support members 102, 104, 106, and 108. The anchors are formed by bending the support members to an angle that will result in a deployed anchor engaging the air passageway wall by piercing it approximately perpendicularly. In this exemplary embodiment, the bend angle is approximately a right angle. Anchor ends 122, 124, 126, and 128 may be shaped to promote piercing the air passageway wall.

Obstructing member 110 is carried on the support structure 101, and includes a flexible membrane open in the proximal direction and which may be formed of silicone or polyurethane, for example. The obstructing member 110 is secured to the central support structure 109, and may be additionally secured to the support members at its larger diameter 91. It may be secured by adhesive, or other manner known in the art. Obstructing member 110 may be loosely carried on support members 102, 104, 106, and 108, such that it expands on inhalation to form a seal against a wall of the air passageway, and contracts on exhalation to allow air and mucociliary transport from the collapsed lung. This provides a one-way valve function.

FIG. 6 is a partial section view of the device of FIG. 5 showing additional detail of the support structure. The linear cross-section view of FIG. 6 exposes the arrangement of support members 106 and 108 in their deployed configuration. The details of support members 102 and 104 are omitted from FIG. 6 for clarity, but are the same as support members 106 and 108. The distal end of obstructing member 110 is carried on central support structure 109. Support members 106 and 108 are shown emanating from central support structure 109, and arranged to loosely support to obstructing member 110 at its larger diameter 91. This allows obstructing member 110 to expand on inhalation and seal at the contact zone 129, and to partially contract on exhalation to allow exhalation of air and mucociliary transport. In an alternative exemplary embodiment, support members 106 and 108 do not actively support obstructing member 110, and the expansion and contraction of obstructing member 110 is governed by its elasticity.

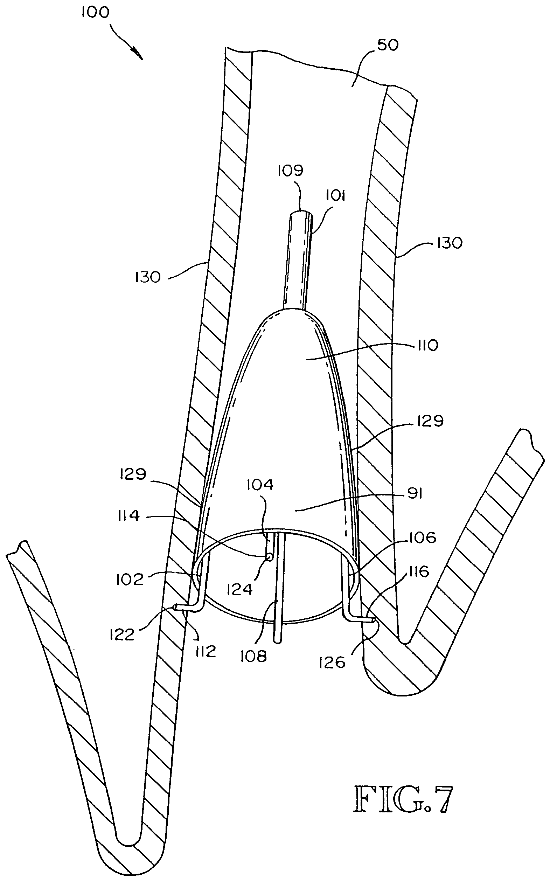

FIG. 7 is a perspective view of the intra-bronchial device of FIG. 5 anchored in an air passageway. Intra-bronchial device 100 is illustrated with anchors 112 and 116 piercing into the air passageway wall 130 of air passageway 50. This anchors the intra-bronchial device 100 in place.

Intra-bronchial device 100 is collapsible for insertion into an internal lumen of a catheter. At least the support members 102, 104, 106, and 108, and the obstructing member 110, may be collapsed. Intra-bronchial device 100 is inserted into the catheter lumen, which is typically already placed in the air passageway 50 as generally illustrated in FIG. 3. Using the stylet, intra-bronchial device 100 is advanced down the catheter lumen into the air passageway 50 to where the device is to be deployed. Once the point of deployment is reached, intra-bronchial device 100 is expelled from the catheter and assumes its deployed shape as illustrated in FIG. 5. Upon deployment, obstructing member 110 expands to form a contact zone 129 with the wall 130 of the air passageway 50 to prevent air from being inhaled into the lung portion to collapse the lung portion. Simultaneously upon deployment, the memory and resiliency of the support members 102, 104, 106, and 108 impart a force on the anchor ends 122, 124, 126, and 128, and urge the anchors 112, 114, 116, and 118 to engage air passageway wall 130 by piercing. The anchors pierce into and become embedded in the wall 130 of the air passageway 50, preferably without projecting through the wall 130. Stops may be incorporated into the anchors to limit piercing of the wall 130. For example, the bend between the support member and the anchor may form a stop.

The preclusion of air from being inhaled into the lung portion may be terminated by eliminating the obstructing effect of intra-bronchial device 100. The preclusion of air by the exemplary embodiment illustrated in FIGS. 5-7 may be eliminated by releasing anchors 112, 114, 116, and 118 from the air passageway wall 130. The anchors may be released by inserting a catheter into air passageway 50 in proximity to intra-bronchial device 100. A retractor device, such as biopsy forceps, capable of gripping a portion of intra-bronchial device 100 is inserted in the catheter. The forceps are used to engage a portion of the support structure 101 of intra-bronchial device 100, and draw it toward the catheter. The drawing action releases anchors 112, 114, 116, and 118 from air passageway wall 130. The intra-bronchial device 110 is then drawn into the catheter with the forceps, causing the support structure 101 and obstructing member 110 to collapse. The collapsed device 100 now fully enters the catheter lumen for removal from the patient. Alternatively, the obstructing effect may be eliminated by grabbing the obstructing member 110, releasing it from the support structure 101, and removing obstructing member 110 from the patient.

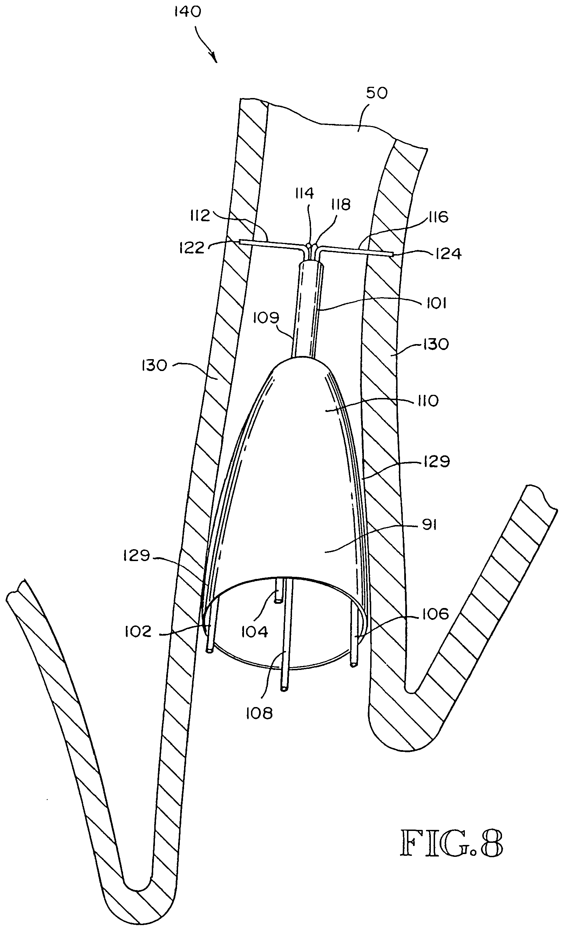

FIG. 8 illustrates an intra-bronchial device, with anchors carried distally on the central support structure, fully deployed in an air passageway in accordance with an alternative exemplary embodiment of the disclosure. The anchors 112, 114, 116, and 118 of intra-bronchial device 140 are carried on portions of support members 102, 104, 106, and 108 distal of the central support structure 109. The support members are gathered together and carried by the central support structure 109. Other than the anchors 112, 114, 116, and 118 being formed and carried on distal portions of support members 102, 104, 106, and 108, intra-bronchial device 140 is substantially similar in construction, operation, and removal as the intra-bronchial device 100 of FIG. 5.

When intra-bronchial device 140 is compressed for insertion into the catheter lumen for placement in the air passageway, the anchors 112, 114, 116, and 118 are collapsed into a first configuration. In the first configuration, the anchor ends 122, 124, 126, and 128 are moved toward obstructing member 110, and anchors 112, 114, 116, and 118 thereby folded toward obstructing member 110. When intra-bronchial device 100 is deployed from the catheter lumen, the memory and resiliency of the support members 102, 104, 106, and 108 impart a force that moves the anchors 112, 114, 116, and 118 into a second configuration to engage air passageway wall 130. This is the deployed configuration illustrated in FIG. 8. For removal, drawing intra-bronchial device 140 toward the catheter causes the anchor ends 122, 124, 126, and 128 to move away from obstructing member 110 to a third configuration. Anchors 112, 114, 116, and 118 are thereby folded away from obstructing member 110 and are released from engagement with air passageway wall 130 for removal of the intra-bronchial device 140. In an alternative exemplary embodiment, the anchors 112, 114, 116, and 118 may be formed on additional support members carried by central support structure 109, instead of being formed from distal portions of support members 102, 104, 106, and 108.

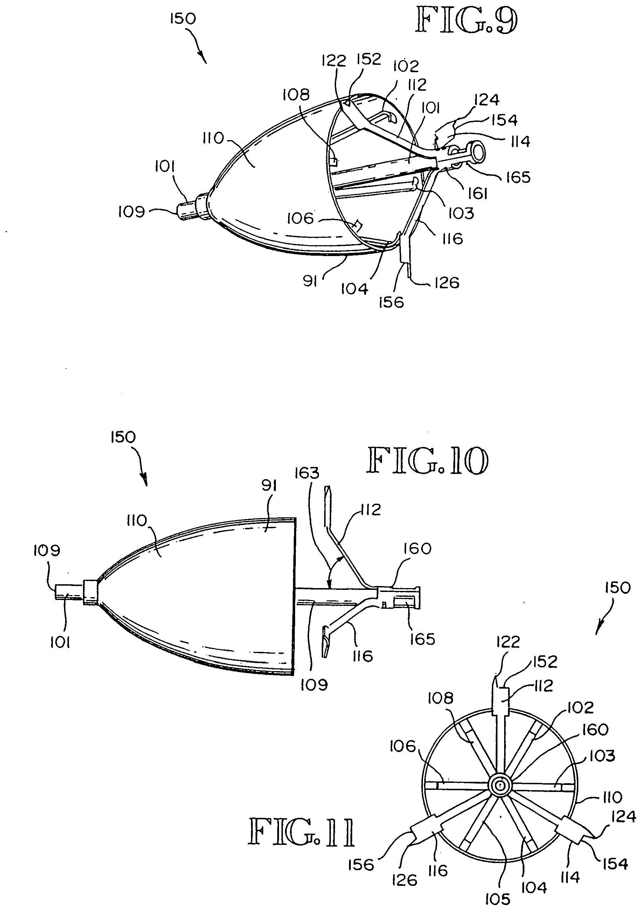

FIGS. 9-11 illustrate an intra-bronchial device, with proximal anchors carried on the central support structure, in accordance with an alternative exemplary embodiment of the disclosure. FIG. 9 is a perspective view, FIG. 10 is a side view, and FIG. 11 is an end view of the device. Intra-bronchial device 150 is generally similar in construction, operation, placement, and removal to the intra-bronchial device 100 of FIG. 5. Its structure has six support members and three anchors, with anchor stops. Anchors 112, 114, and 116 include stops 152, 154, and 156, respectively. Intra-bronchial device 150 also includes an anchor base 160, an anchor base aperture 165, anchor base angle 163, and additional support members 103 and 105.

Central support structure 109 extends both proximal and distal of obstructing member 110, and carries anchor base 161 proximal of obstructing member 110, carries anchors 112, 114, and 116, and includes anchor base aperture 165. The linear plane of anchors 112, 114, and 116 intersect anchor base 161 at anchor base angle 163. Anchor base angle 163 is selected to optimize anchor deployment force and anchor release. Stops 152, 154, and 156 include a flat area to limit the piercing of the air passageway wall by anchor ends 122, 124, and 126. In alternative embodiments, the stops can be any configuration or shape known to those skilled in the art to limit the piercing.

In operation, when intra-bronchial device 150 is compressed for insertion into the catheter lumen for placement in the air passageway, anchors 112, 114, and 116 are collapsed into a first configuration. In the first configuration, the anchor ends 122, 124, and 126 are moved toward obstructing member 110, thereby decreasing anchor base angle 163 and folding anchors 112, 114, and 116 toward obstructing member 110. The anchor ends and the anchors may be moved by sliding a catheter or hollow member over anchor base 161 and toward obstructing member 110. When intra-bronchial device 150 is deployed from the catheter lumen, the memory and resiliency of the anchors 112, 114, and 116, anchor angle 163, and anchor base 161 impart a force that moves the anchor members into a second configuration, which is the deployed configuration, to engage air passageway wall 130. The second or deployed configuration is illustrated in FIGS. 9-11. Stops 152, 154, and 156 limit the piercing of the air passageway wall by anchor ends 122, 124, and 126.

For removal, a retractor device is deployed from a catheter to engage anchor base 161 and restrain intra-bronchial device 150. The retractor device may be a biopsy forceps to engage anchor base 161, or a hooked device to engage anchor base aperture 165. A catheter is then moved distally over anchor base 161, and in contact with anchors 112, 114, and 116. The catheter is further moved against anchors 112, 114, and 116, while intra-bronchial device 150 is restrained at anchor base 161. This releases the anchors 112, 114, and 116 from the air passageway wall. This collapses the anchors into to the first configuration for removal. Intra-bronchial device 150 is then further drawn into the catheter by pulling on the retractor device used to engage anchor base 161. This collapses support structure 101 and obstructing member 110 so that they may be fully drawn into the catheter. Once drawn into the catheter, intra-bronchial device 160 may be removed from the air passageway and the patient.

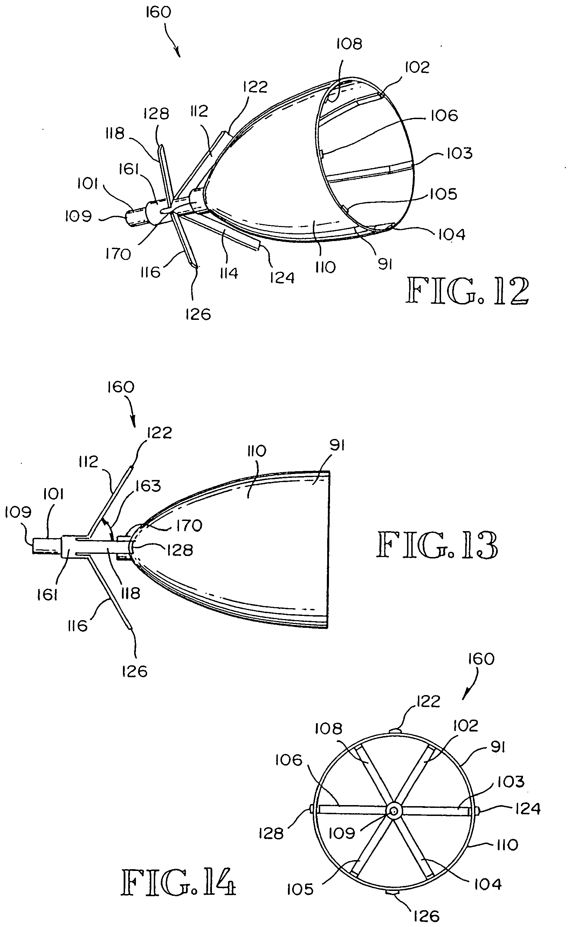

FIGS. 12-14 illustrate an intra-bronchial device, with distal friction anchors carried on the central support structure, in accordance with an embodiment of the invention. FIG. 12 is a perspective view, FIG. 13 is a side view, and FIG. 14 is an end view. Intra-bronchial device 160 is generally similar in construction, placement, and operation to the intra-bronchial device 150 of FIGS. 9-11. Intra-bronchial device 160 is removed in the manner described in conjunction with FIG. 7. However, Intra-bronchial device 160 differs from intra-bronchial device 150 in that the structure includes four distal anchors with anchor ends 122, 124, 126, and 128 shaped into pads that deform and frictionally engage the air passageway wall to more positively anchor intra-bronchial device 160 without piercing. The structure also includes an obstructing member support base 170.

Central support structure 109 extends distal of obstructing member 110, and carries anchor base 161 distal of obstructing member 110. Anchor base 161 carries anchors 112, 114, 116, and 118. The linear plane of anchors 112, 114, 116, and 118 intersects anchor base 161 at anchor angle 163. Anchor angle 163 is selected to optimize anchor deployment force and anchor release. The anchors 112, 114, 116, and 118, and anchor base 161 may be constructed by laser cutting a single piece of hypodermic tubing lengthwise to form the anchors 112, 114, 116, and 118, and then bending the anchors to form anchor angle 163. Anchor base 161 is secured to central support structure 109. Support members 102, 103, 104, 105, 106, and 108, and the obstructing member support member base 170 may be constructed in a like manner. Obstructing member 110 is secured to the obstructing member support base 170, and alternatively to support members 102, 103, 104, 105, 106, and 108. The assembly of obstructing member 110 and support base 170 is secured to central support structure 109. Central support structure 109 may extend proximal of support member base 170 to provide a surface for gripping the intra-bronchial device 160 for removal, and may include an aperture to be engaged by a hooked device.

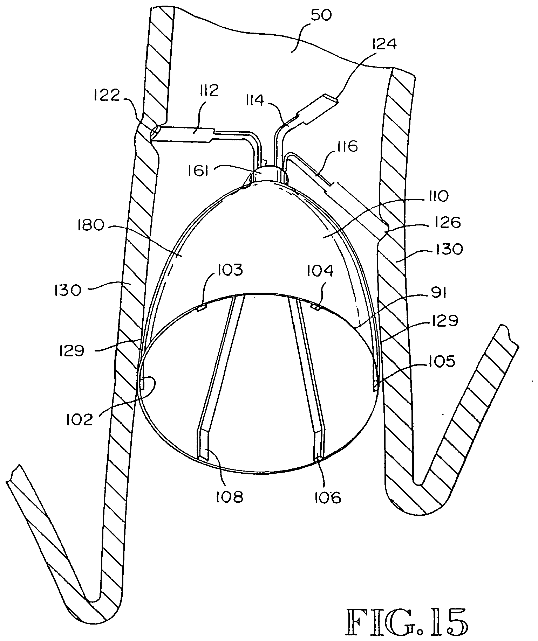

FIG. 15 is a perspective view an exemplary intra-bronchial device similar to that of FIGS. 12-14 anchored in an air passageway. It illustrates pad-shaped anchor ends 122-128 of intra-bronchial device 180 deforming and frictionally engaging air passageway wall 130.

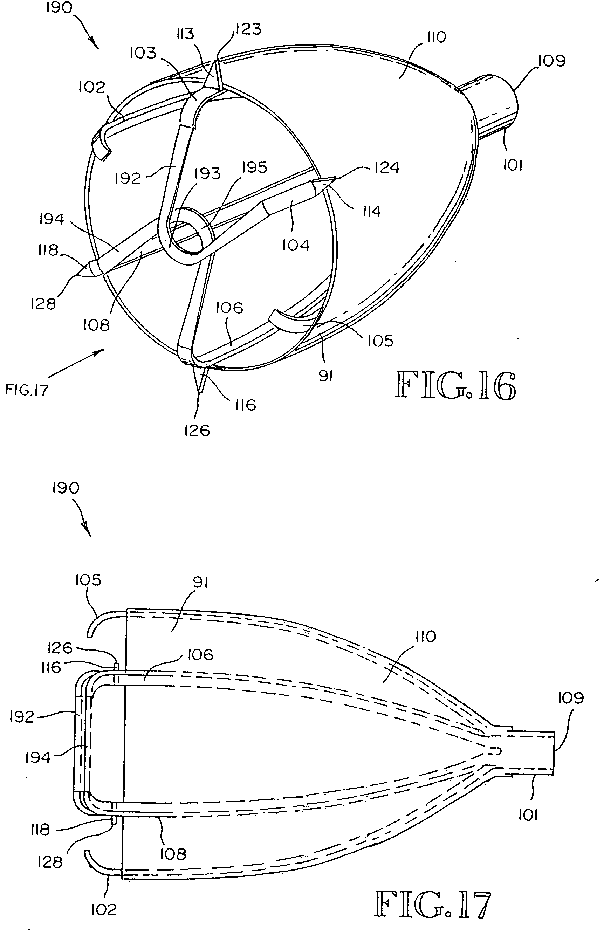

FIGS. 16 and 17 illustrate an exemplary removable intra-bronchial device with proximal anchors carried on a peripheral portion of a plurality of support structure members in accord with the present disclosure. FIG. 16 is a perspective view, as the device would appear when fully deployed in an air passageway. FIG. 17 is a side view of FIG. 16. In an exemplary embodiment, the support structure 101 of intra-bronchial device 190 includes six support members, with two opposing pairs of support members carrying anchors and each member of a pair being joined together by a retracting member. Intra-bronchial device 190 includes a support structure 101 having a central support structure 109 and support members 102, 103, 104, 105, 106, and 108; four anchors 113, 114, 116, and 118 having anchor ends 123, 124, 126, and 128, respectively; two "U" shaped retracting members 192 and 194 having an apex 193 and 195, respectively; and obstructing member 110.

Intra-bronchial device 190 is generally similar in construction, operation, placement, and removal to the intra-bronchial device 150 of FIG. 9. Support structure 101 is a tubular member, preferably hypodermic needle tubing, or stainless steel, Nitinol, or other suitable material having a memory of its original shape and resiliency to return the material to that shape. Support members 102, 103, 104, 105, 106, and 108, and central support structure 109 may be formed by laser cutting a single piece of hypodermic needle tubing lengthwise, and bending the support members to a required shape. Support members 102, 103, 104, 105, 106, and 108 are generally similar to each other. Anchors 113, 114, 116, and 118 are disposed on support members 103, 104, 106, and 108, respectively, in any manner available in the art. Anchors 113-118 are disposed on support members 103, 104, 106, and 108 to be located proximally of obstructing member 110, and to engage an air passageway wall when intra-bronchial device 190 is deployed.

"U" shaped retracting member 192 is coupled to support members 103 and 104, and "U" shaped retracting member 194 is coupled to support members 106 and 108. "U" shaped retracting members 192 and 194 may be constructed of any material suitable for use within a patient, and may or may not be resilient as required by the particular embodiment. When intra-bronchial device 190 is fully deployed in an air passageway, the "U" shaped retracting members 192 and 194 are arranged opposite each other, and they partially overlap, with the apex of one lying within a space bounded by the "U" shape of the other member. In the deployed configuration, increasing the distance between apex 193 and apex 195 moves support member pairs 103-104 and 106-108 centrally.

In operation, when intra-bronchial device 190 is compressed for insertion into a catheter lumen and placement in an air passageway, support members 102, 103, 104, 105, 106, and 108 are collapsed centrally into a first configuration. This causes the anchor ends 123-124, and 126-128 to move centrally.

When intra-bronchial device 190 is deployed from the catheter lumen, the memory and resiliency of the support member pairs 103,104 and 106,108 impart a force that moves the anchors 113 and 114, and 116 and 118, and their anchor ends 123 and 124, and 126 and 128 into a second configuration, which is the deployed configuration to engage air passageway wall. In addition, deployment of intra-bronchial device 190 may include a step of forcibly decreasing the distance between apexes 193 and 195 to forcibly move the anchors 113 and 114, and 116 and 118 into engagement with the wall of the air passageway. While the anchors 113 and 114, and 116 and 118 of this exemplary embodiment do not include stops, the expansive or peripheral movement of the anchors will be limited by obstructing member 90. This may limit the piercing of the air passageway wall by anchors 113 and 114, and 116 and 118.

In an alternative exemplary embodiment, support member pairs 103,104 and 106,108 may be compressed for insertion into a catheter lumen by a device that increases the distance between apex 193 and apex 195. Such a device could be a tool with spreading jaws, or a tapered member inserted between the apexes. The device could be left in engagement after insertion into the catheter, and then withdrawn to allow support member pairs 103-104 and 106-108 to move apart and engage their anchors into the wall of the air passageway.

For removal, a retractor device is deployed from a catheter lumen to engage apex 193 and 195, and restrain intra-bronchial device 190. The retractor device may be any device that fits into the space defined by apexes 193 and 195 when the intra-bronchial device 190 is in its fully deployed configuration. The retractor device is used to increase the distance between apexes 193 and 195 until anchors 113-114 and 116-118, and anchor ends 123-124 and 126-128 are released from the air passageway wall. This collapses the anchors into to the first configuration for removal. Intra-bronchial device 190 is then further collapsed, and drawn into the catheter by pulling on the retractor device. This additionally collapses support structure 101 and obstructing member 110 into the first position so that they may be fully drawn into the catheter. Once drawn into the catheter, intra-bronchial device 190 may be removed from the air passageway and the patient.

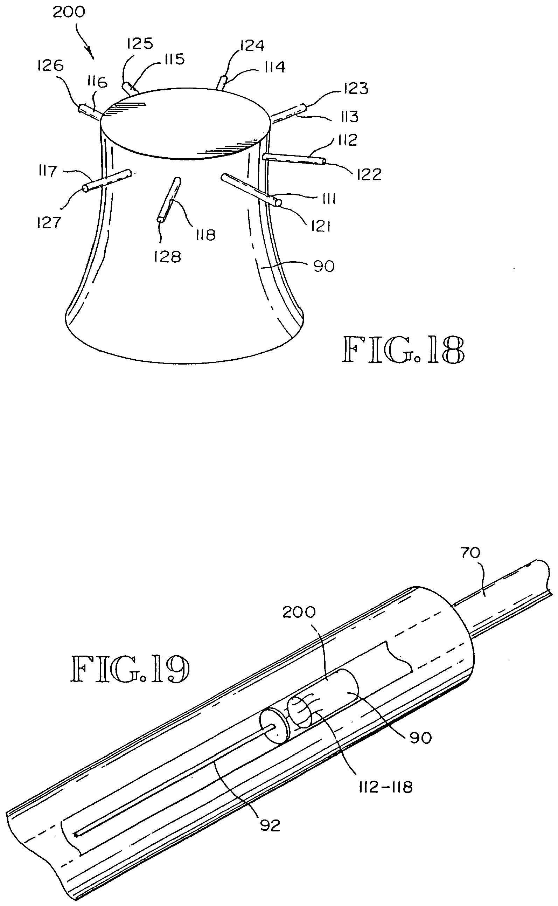

FIG. 18 is a perspective view of an exemplary intra-bronchial device 200 with anchors carried on an obstructing member as the device would appear when fully deployed in an air passageway, in accordance with an alternative exemplary embodiment of the disclosure. Intra-bronchial device 200 includes an obstructing member 90, anchors 111, 112, 113, 114, 115, 116, 117, and 118 (hereafter collectively referred to as anchors 111-118), and anchor ends 121, 122, 123, 124, 125, 126, 127, and 128(hereafter collectively referred to as anchor ends 121-128).

Obstructing member 90 may be a single piece made from a collapsible, resilient material, such as silicone, polyurethane, rubber, or foam, and typically will be collapsible to at least one-half of its expanded size. In an alternative embodiment, obstructing member 90 may include multiple pieces, some being of collapsible material. In a further alternative embodiment, obstructing member 90 may include a membrane carried by a support structure such as described in conjunction with FIGS. 5-17.

Anchors 111-118 comprise material having memory of an original shape, and resiliency to return the material to that shape, and typically have a diameter small enough that penetration through an air passageway wall may not adversely effect a patient. Anchors 111-118 may be 0.076 mm (0.003-inch) diameter 316 stainless steel with a wire spring temper, Nitinol, or other resilient material. Anchor ends 121-128 may be shaped to promote or control piercing of the air passageway wall. In an alternative embodiment, the length of the anchors 111-118 may be limited to allow the anchors 111-118 to penetrate into but not through the air passageway wall. In the preferred exemplary embodiment illustrated in FIG. 18, the anchors include four pieces of material pushed through obstructing member 90. The four pieces would lie in approximately the same cross-sectional plane, and cross each other at approximately the centerline of obstructing member 90, with approximately equal portions of the anchor material projecting from opposite sides of the obstructing member 90. In this embodiment, for example, anchors 112 and 116 would be opposite portions of a single piece of material. Anchors 111-118 may be secured to control their position. For example, a centerline opening may be made in obstructing member 90 exposing the several pieces of anchoring material. The several pieces of material could them be joined together, or to obstructing member 90, at a location within the centerline opening by an adhesive, crimping, welding, or other method of mechanically joining materials known to those in the art.

In an alternative exemplary embodiment, the anchors may be formed by individual pieces of material. The individual pieces of material may be coupled to obstructing member 90 either at its periphery, or within its periphery.

FIG. 19 is a partial longitudinal sectional view of the intra-bronchial device of FIG. 18 collapsed and located into a delivery catheter lumen for placement in an air passageway to collapse a lung portion associated with the air passageway, in accordance with the present disclosure. Intra-bronchial device 200 is generally placed in an air passageway as described in FIGS. 2 and 3.

More specifically, intra-bronchial device 200 is collapsed and placed into delivery catheter lumen 70. Obstructing member 90 is collapsed into approximately a cylindrical shape. Anchors 111-118 are collapsed to a position in proximity to or against the outer periphery of collapsed obstructing member 90. Intra-bronchial device 200 is inserted into catheter lumen 70, the distal end of which is typically already placed in the air passageway 50 as generally illustrated in FIG. 3. Using stylet 92, intra-bronchial device 200 is advanced through the catheter lumen 70 into the air passageway to where it is to be deployed. Once the point of deployment is reached, intra-bronchial device 200 is expelled from catheter lumen 70, and assumes a deployed shape as illustrated in FIG. 18.

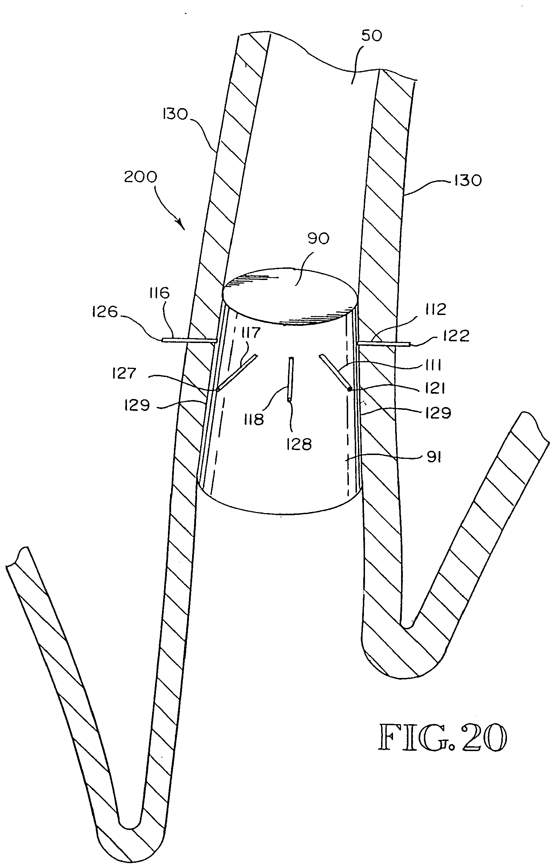

FIG. 20 is a perspective view of the intra-bronchial device of FIG. 18 in its fully deployed and anchored state in an air passageway, in accordance with the present invention. Intra-bronchial device 200 is illustrated after having been expelled from the catheter lumen in substantially the manner described in conjunction with FIG. 3, and having deployed anchors 112 and 116 by piercing into and through air passageway wall 130 of air passageway 50. The piercing engages the air passageway wall and anchors intra-bronchial device 200 within the air passageway 50.

The resiliency of obstructing member 90 imparts a force to expand the obstructing member 90 from the collapsed state to a deployed state. In its deployed state, obstructing member 90 forms a contact zone 129 with the wall 130 of air passageway 50 preventing air from being inhaled into the lung portion. The resiliency of the anchor members 111-118 moves them from their collapsed state illustrated in FIG. 19 to their deployed state. The resiliency of obstructing member 90 may assist anchor members 111-118 in deployment. In the alternative embodiment where the length of anchors 111-118 is limited to allow the anchors 111-118 to penetrate into but not through the air passageway wall, the anchors penetrate the air passageway wall 150 in the manner illustrated in FIG. 7.

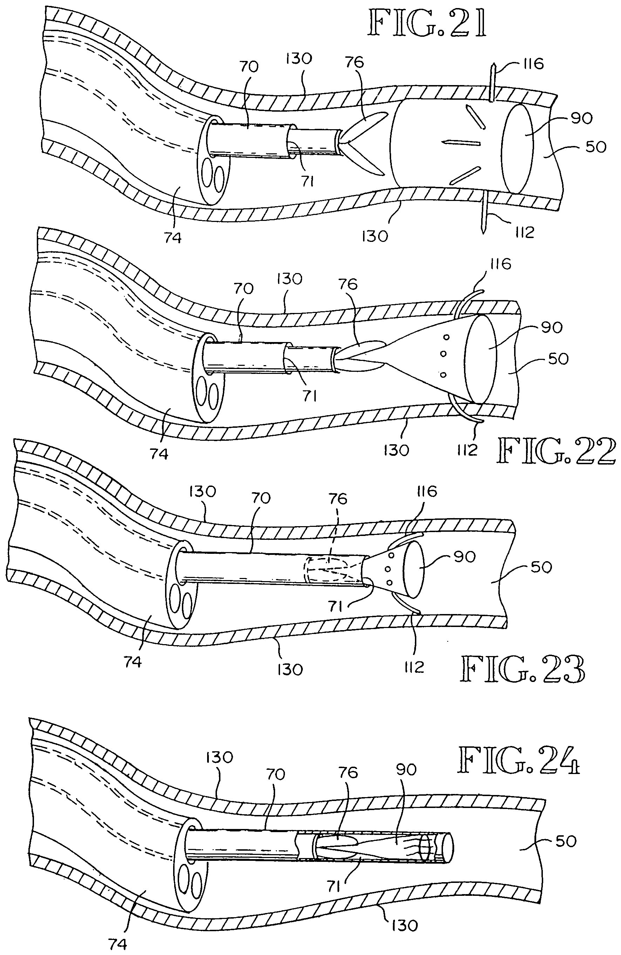

FIGS. 21-24 are side views showing an exemplary embodiment of the present invention for removing the intra-bronchial device 200 from air passageway 50. The preclusion of air from being inhaled into the lung portion may be terminated by eliminating the obstructing effect of intra-bronchial device 200. The preclusion of air by the embodiment illustrated in FIG. 18 may be eliminated by releasing anchors 111-118 from the air passageway wall 130.

A bronchoscope 74 is placed in proximity to intra-bronchial device 200 in the air passageway 50. A catheter 70 having an internal lumen 71 is fed into the bronchoscope 74 and advanced to the proximal end of the intra-bronchial device 200. A retractor device, such as biopsy forceps 76, capable of gripping a portion of intra-bronchial device 200, is inserted in the catheter 70 of the bronchoscope 74 and advanced to the intra-bronchial device 200. The jaws of the forceps 76 are used to engage a portion of the obstructing member 90. The engagement may collapse a portion of obstructing member 90. The engagement with the obstructing member 90 is maintained and obstructing member 90 is drawn toward the catheter lumen 71 by the forceps 76. The drawing action releases anchors 111-118 from air passageway wall 130. The intra-bronchial device 200 is then drawn into the catheter lumen 71 with the forceps 76. The collapsed device 200 now fully enters the catheter lumen 71 for removal from the patient.

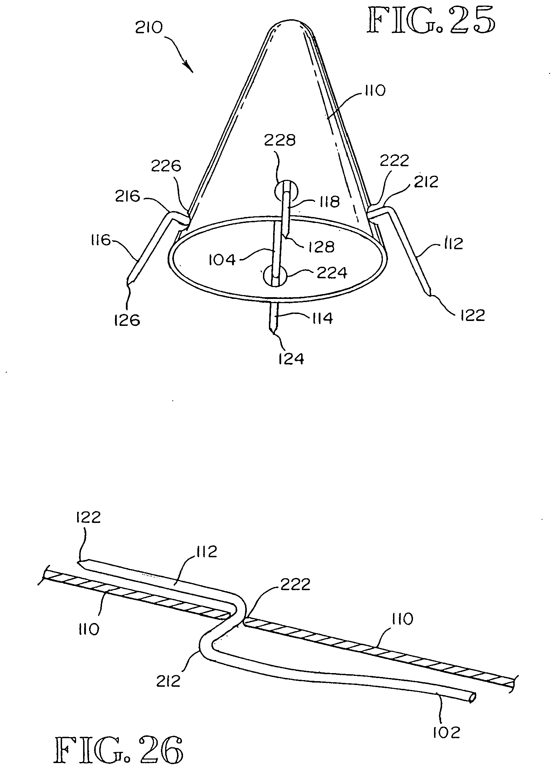

FIG. 25 is a perspective view of an exemplary intra-bronchial device with anchors projecting from a periphery of an obstructing member as the device would appear when fully deployed, in accordance with an alternative exemplary embodiment of the present disclosure. The intra-bronchial device 210 includes support members 102, 104, 106, and 108; an obstructing member 110; "s" shaped bends 212, 214, 216, and 218; and anchors 112, 114, 116, and 118.

The support members 102, 104, 106, and 108 form a support structure carrying obstructing member 110, and include anchors 112, 114, 116, and 118; and anchor ends 122, 124, 126, and 128, respectively. The support members 102, 104, 106, and 108 may be tubular members, and are preferably hypodermic needle tubing. Support members 102, 104, 106, and 108 form a support structure by being joined together at a location toward the distal portion of intra-bronchial device 210. They may be joined by a mechanical method, such as by crimping, or by other methods such as adhesive or welding. In an alternative embodiment, two support members may be formed from a single piece of material by bending it in the middle. Support members 102, 104, 106, and 108 are generally similar to each other. The support members are preferably formed of stainless steel, Nitinol, or other suitable material having a memory of its original shape, and resiliency to return the material to that shape.

Anchors 112, 114, 116, and 118 are extensions of support members 102, 104, 106, and 108. The anchors are formed by forming "s" shaped bends 212, 214, 216, and 218 in proximal portions of the support members. Anchor ends 122, 124, 126, and 128 may be shaped to promote piercing the air passageway wall.

The obstructing member 110 is carried on the support structure formed by support members 102, 104, 106, and 108. Obstructing member 110 includes a flexible membrane open in the proximal direction and which may be formed of silicone or polyurethane, for example. The obstructing member 110 includes openings 222, 224, 226, and 228 sized to sealingly admit the "s" shaped bends 212, 214, 216, and 218 of support members 102, 104, 106, and 108, respectively. FIG. 26 illustrates the placement and securing of the obstructing member 110 to support member 102 at "s" bend 212. Obstructing member 110 is fitted over the anchor end 122 and anchor 112 at opening 222. Obstructing member 110 engages the peripheral apex of the "s" shaped bend 212, and thus secures it. The obstructing member 110 is placed and secured to the other "s" bends 214, 216, and 218 in a similar manner. Obstructing member 110 may be loosely carried on support members 102, 104, 106, and 108 such that it expands on inhalation to form a seal against a wall of the air passageway, and contracts on exhalation to allow air and mucociliary transport from the collapsed lung. This provides a one-way valve function.

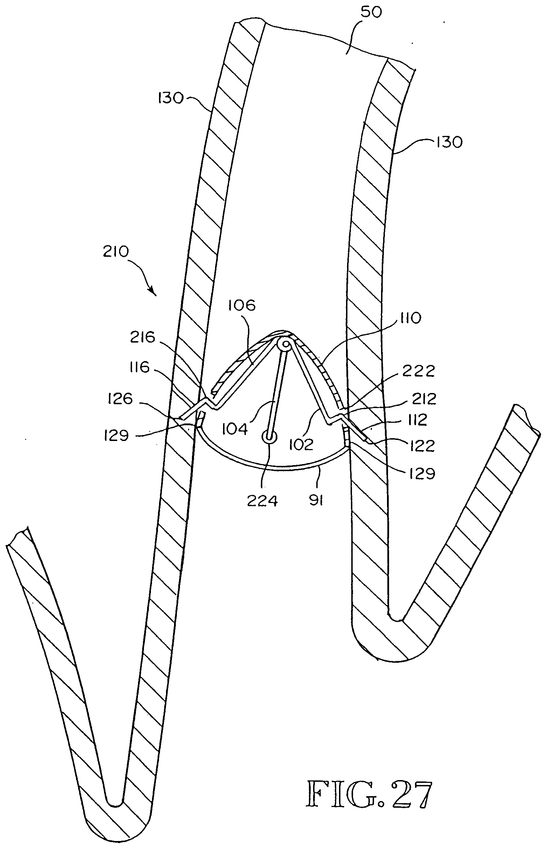

FIG. 27 is a perspective view of the intra-bronchial device of FIG. 25 fully deployed and anchored in an air passageway, in accordance with the present disclosure. Intra-bronchial device 210 is illustrated after having been expelled from the catheter lumen in substantially the manner described in conjunction with FIG. 3, and having deployed anchors 112, 114, 116, and 118 by piercing into air passageway wall 130 of air passageway 50. The piercing engages the air passageway wall and anchors intra-bronchial device 210 within the air passageway 50.

Deploying obstructing member 210 is much like opening an umbrella. Upon deployment, the memory and resiliency of the support members 102, 104, 106, and 108, expand obstructing member 210. The expanded obstructing member 210 forms a contact zone 129 with the wall 130 of the air passageway 50 to prevent air from being inhaled into the lung portion to collapse the lung portion. Simultaneously upon deployment, the memory and resiliency of the support members 102, 104, 106, and 108 impart a force on the anchor ends 122, 124, 126, and 128, and urge the anchors 112, 114, 116, and 118 to engage air passageway wall 130 by piercing. The anchors pierce into and become embedded in the wall 130 of the air passageway 50, preferably without projecting through the wall 130. Stops may be incorporated into the anchors to limit piercing of the wall 130. For example, the "s" bends 212, 214, 216, and 218 may form a stop.

The preclusion of air from being inhaled into the lung portion may be terminated by eliminating the obstructing effect of intra-bronchial device 210. The preclusion of air by the embodiment illustrated in FIGS. 25-27 may be eliminated by releasing anchors 112, 114, 116, and 118 from the air passageway wall 130. The anchors are released and the intra-bronchial device 210 is removed from air passageway 50 in substantially the same manner described in conjunction with FIGS. 7, and 21-24. The forceps are used to engage a portion of intra-bronchial device 210, and maneuvered to release anchors 112, 114, 116, and 118 from the air passageway wall 130. Intra-bronchial device 210 is then drawn into the catheter for removal from the patient. Alternatively, the obstructing effect may be eliminated by engaging the obstructing member 210, releasing it from the support members 102, 104, 106, and 108, and drawing obstructing member 110 into the catheter for removal from the patent.

Although the present invention has been described in considerable detail with reference to certain preferred embodiments, other embodiments are possible. Therefore, the scope of the appended claims should not be limited to the description of the embodiments contained herein. It is intended that the invention resides in the claims hereinafter appended.

## Claims

1. An intra-bronchial device (160) comprising:
an obstructing member support member base (170);
a central support structure (109) connected to the obstructing member support member base (170) and extending distal therefrom;
an obstructing member (110) secured to six support members (102, 103, 104, 105, 106, 108), the six support members (102, 103, 104, 105, 106, 108) connected to the obstructing member support member base (170) and extending proximally from the obstructing member support member base (170) and having proximal ends that bend inwardly towards a center of the obstructing member (110);
an anchor base (161) carried on the central support structure (109) distal of the obstructing member (110); and four distal anchors (112, 124, 126, 128) connected to the anchor base (161) and having ends (122, 124, 126, 128) having pads.

2. The intra-bronchial device (160) of claim 1, wherein the anchors (112, 124, 126, 128) and anchor base (161) are constructed by laser cutting a single piece of hypodermic tubing lengthwise to form the anchors (112, 124, 126, 128), and then bending the anchors.

3. The intra-bronchial device (106) of claim 1, wherein the central support structure (109) includes an aperture to be engaged by a hooked device.

4. The intra-bronchial device (160) of claim 1, wherein the obstructing member (110) is secured to the central support structure (109) via obstructing member support member base (170).

5. The intra-bronchial device (160) of claim 1, wherein a linear plane of the anchors (112, 114, 116, 118) intersects the anchor base (161) at an anchor angle (163).

6. The intra-bronchial device (160) of claim 1, wherein the anchor angle (163) is selected to optimize anchor deployment force and anchor release.

7. The intra-bronchial device (160) of claim 1, wherein the support members (102, 103, 104, 105, 106, 108) and the obstructing member support member base (170) are constructed by laser cutting a single piece of hypodermic tubing lengthwise to form the support members (102, 103, 104, 105, 106, 108), and then bending the support members.

8. The intra-bronchial device (160) of claim 1, wherein the anchor ends (122, 124, 126, 128) frictionally engage an air passageway wall.

## Patentansprüche

1. Intrabronchialvorrichtung (160), umfassend:
eine Sperrelementstützelementbasis (170);
eine mittige Stützstruktur (109), die mit der Sperrelementstützelementbasis (170) verbunden ist und sich distal davon erstreckt;
ein Sperrelement (110), das an sechs Stützelementen (102, 103, 104, 105, 106, 108) befestigt ist, wobei die sechs Stützelemente (102, 103, 104, 105, 106, 108) mit einer Sperrelementstützelementbasis (170) verbunden sind und sich proximal aus der Sperrelementstützelementbasis (170) erstrecken und proximale Enden aufweisen, die sich nach innen in Richtung einer Mitte des Sperrelements (110) biegen;
eine Ankerbasis (161), die an der mittigen Stützstruktur (109) distal des Sperrelements (110) getragen wird; und
vier distale Anker (112, 124, 126, 128), die mit der Ankerbasis (161) verbunden sind und
Enden (122, 124, 126, 128) mit Bäuschen aufweisen.

2. Intrabronchialvorrichtung (160) nach Anspruch 1, wobei die Anker (112, 124, 126, 128) und die Ankerbasis (161) durch Laserschneiden eines einzelnen Stücks eines Injektionsschlauchs der Länge nach konstruiert sind, um die Anker (112, 124, 126, 128) auszubilden, und anschließend die Anker zu biegen.

3. Intrabronchialvorrichtung (106) nach Anspruch 1, wobei die mittige Stützstruktur (109) eine Öffnung beinhaltet, die von einer Hakenvorrichtung in Eingriff genommen werden soll.

4. Intrabronchialvorrichtung (160) nach Anspruch 1, wobei das Sperrelement (110) über die Sperrelementstützelementbasis (170) an der mittigen Stützstruktur (109) befestigt ist.

5. Intrabronchialvorrichtung (160) nach Anspruch 1, wobei eine lineare Ebene der Anker (112, 114, 116, 118) die Ankerbasis (161) an einem Ankerwinkel (163) überschneidet.

6. Intrabronchialvorrichtung (160) nach Anspruch 1, wobei der Ankerwinkel (163) ausgewählt ist, um die Ankerentfaltungskraft und die Ankerfreigabe zu optimieren.

7. Intrabronchialvorrichtung (160) nach Anspruch 1, wobei die Stützelemente (102, 103, 104, 105, 106, 108) und die Sperrelementstützelementbasis (170) durch Laserschneiden eines einzelnen Stücks eines Injektionsschlauchs der Länge nach konstruiert sind, um die Stützelemente (102, 103, 104, 105, 106, 108) auszubilden und anschließend die Stützelemente zu biegen.

8. Intrabronchialvorrichtung (160) nach Anspruch 1, wobei die Ankerenden (122, 124, 126, 128) eine Atemwegswand reibschlüssig in Eingriff nehmen.

## Revendications

1. Dispositif intrabronchique (160) comprenant :
une base d'élément de support d'élément d'obstruction (170) ;
une structure de support centrale (109) reliée à la base d'élément de support d'élément d'obstruction (170) et s'étendant distalement à partir de celle-ci ;
un élément d'obstruction (110) fixé à six éléments de support (102, 103, 104, 105, 106, 108), les six éléments de support (102, 103, 104, 105, 106, 108) étant reliés à la base d'élément de support d'élément d'obstruction (170) et s'étendant de façon proximale à partir de la base d'élément de support d'élément d'obstruction (170) et ayant des extrémités proximales qui se courbent vers l'intérieur vers un centre de l'élément d'obstruction (110) ;
une base d'ancrage (161) portée sur la structure de support centrale (109) distale de l'élément d'obstruction (110) ; et
quatre ancrages distaux (112, 124, 126, 128) reliés à la base d'ancrage (161) et ayant des extrémités (122, 124, 126, 128) ayant des coussinets.

2. Dispositif intrabronchique (160) selon la revendication 1, dans lequel les ancrages (112, 124, 126, 128) et la base d'ancrage (161) sont construits par découpe au laser d'une seule pièce de tubulure hypodermique dans le sens de la longueur pour former les ancrages (112, 124, 126, 128), puis pour courber les ancrages.

3. Dispositif intrabronchique (106) selon la revendication 1, dans lequel la structure centrale de support (109) comporte une ouverture afin de venir en prise avec un dispositif accroché.

4. Dispositif intrabronchique (160) selon la revendication 1, dans lequel l'élément d'obstruction (110) est fixé à la structure de support centrale (109) par l'intermédiaire de la base d'élément de support d'élément d'obstruction (170).

5. Dispositif intrabronchique (160) selon la revendication 1, dans lequel un plan linéaire des ancrages (112, 114, 116, 118) coupe la base d'ancrage (161) selon un angle d'ancrage (163).

6. Dispositif intrabronchique (160) selon la revendication 1, dans lequel l'angle d'ancrage (163) est choisi pour optimiser la force de déploiement de l'ancrage et la libération de l'ancrage.

7. Dispositif intrabronchique (160) selon la revendication 1, dans lequel les éléments de support (102, 103, 104, 105, 106, 108) et la base de l'élément de support d'élément d'obstruction (170) sont construits par découpe au laser d'une seule pièce de tubulure hypodermique dans le sens de la longueur pour former les éléments de support (102, 103, 104, 105, 106, 108), puis pour courber les éléments de support.

8. Dispositif intrabronchique (160) selon la revendication 1, dans lequel les extrémités d'ancrage (122, 124, 126, 128) viennent en prise par friction avec une paroi de passage d'air.
